# EUROPÄISCHE PATENTSCHRIFT

(11) **EP 0 408 856 B1**
(45) Veröffentlichungstag und Bekanntmachung des Hinweises auf die Patenterteilung: **01.09.1993**
(21) Anmeldenummer: 90109417.7
(22) Anmeldetag: 18.05.1990
(51) Int. Cl.: C07C 43/11, C07C 43/23, C07C 41/03, C08G 65/28

(54) **Verfahren zur Herstellung von alkylenoxid- und dioxan-1,4-armen nicht-ionogenen Tensiden mittels Alkalimetallalkoholat als Katalysator**
Process for the preparation of nonionic surfactants low in alkylene oxides and 1,4-dioxane by means of alkali metal alcoholate catalyst
Procédé pour la préparation de tensio-actifs non-ioniques ayant une faible concentration en oxydes d'alkylènes au moyen d'un alcoolat d'un métal alcalin comme catalyseur

(30) Priorität: 17.07.1989 DE 3923562
(43) Veröffentlichungstag der Anmeldung: 23.01.1991
(73) Patentinhaber: HÜLS AKTIENGESELLSCHAFT, 45764 Marl (DE)
(72) Erfinder: Schröer, Egbert, D-4270 Dorsten (DE); Schulze, Klaus, Dr., D-4358 Haltern (DE); Wienhöfer, Ekkehard, Dr., D-4370 Marl (DE)

(56) Entgegenhaltungen:
- EP-A- 0 026 547
- EP-A- 0 336 263
- US-A- 4 453 023

## Beschreibung

Die Erfindung betrifft die Herstellung von alkylenoxid- und dioxan-1,4-armen nicht-ionogenen Tensiden durch Katalyse mit einem Alkalimetallfettalkoholat sowie die Herstellung des Alkalimetallfettalkoholates.

Aufgrund der meist täglichen Anwendung von Produkten, in denen nicht-ionogene Tenside enthalten sind und auf dem Hintergrund möglicher toxikologischer Gefährdungen durch größere Restmengen an Alkylenoxiden und Dioxan-1,4 in diesen Tensiden, ist es notwendig, alkylenoxid- und dioxan-1,4-arme Produkte zur Verfügung zu stellen.

Üblicherweise erfolgt die Herstellung nicht-ionogener Tenside durch Katalyse mit Na- bzw. K-Ionen, welche vorzugsweise als wäßrige NaOH oder KOH bzw. als Na- oder K-methylat den Edukten wie Alkylphenolen, Fettalkoholen, Glycolen, Aminen, Fettsäuren und Ölen zugesetzt werden. Lösungs- und Reaktionswasser werden nach der Inertisierung mit Stickstoff entfernt. Anschließend erfolgt die Umsetzung mit Alkylenoxid.

Nach der US-PS 4 453 023 können auch Ba-Alkoholate als Katalysator dienen, die nach dieser US-Druckschrift durch Reaktion des Ba-Metalles mit Ethanol und anschließender Umsetzung z. B. mit Ethylhexanol hergestellt werden können. Das entstehende Bariumethylhexanolat wird durch Vakuumdestillation von Ethanol gereinigt.

Eine ähnliche Katalysatorherstellung beschreibt die EP-PS 0 026 547. Hiernach können Ca-, Sr- oder Ba-Alkoholate durch Reaktion des Ca-, Sr- oder Ba-Metalles mit Ethanol und anschließender Umsetzung mit Decanol hergestellt werden. Das dabei wieder entstehende Ethanol wird nachher durch Anlegen von Vakuum entfernt.

Sowohl das Verfahren zur Herstellung von nicht-ionogenen Tensiden nach der US-PS 4 453 023 als auch der Einsatz von Katalysatoren bei der Herstellung nicht-ionogener Tenside, die nach dem zweistufigen Verfahren durch Umsetzung eines Alkalimetalles mit Ethanol und anschließender Umsetzung dieses Ethanolats mit einem höheren Alkohol hergestellt worden sind, weisen neben einer extremen Kostenungünstigkeit den Nachteil hoher Restmengen an Alkylenoxid (≈ 30 ppm) und Dioxan-1,4 (≈ 120 ppm) auf. Außerdem ist der Einsatz von Metallen zur Alkoholatherstellung aufgrund der Wasserstoffentwicklung nicht unproblematisch und Produkte, die mit Ethylhexanolatkatalysatoren hergestellt worden sind, riechen stark nach Ethylhexanol.

Es bestand daher die Aufgabe, ein Verfahren zur Herstellung alkylenoxid- und dioxan-1,4-armer, nicht-ionogener, oxethylierter Tenside aufzufinden.

Die Aufgabe wurde durch ein Verfahren, das bei der Alkoxylierung der Edukte als Katalysator eine durch direkte Umsetzung von Alkalihydroxid mit Fettalkohol gewonnenes Fettalkoholat verwendet, gelöst.

Gegenstand der Erfindung ist daher ein Verfahren zur Herstellung alkylenoxid- und dioxan-1,4-armer Alkoxidationsprodukte von Alkylphenolen und Fettalkoholen, das dadurch gekennzeichnet ist, daß man als Katalysator durch direkte Umsetzung bei erhöhter Temperatur von Alkalihydroxid mit Fettalkoholen gewonnene Alkalifettalkoholate einsetzt.

Es wurde überraschend gefunden, daß sich durch den Einsatz von erfindungsgemäß hergestellten Katalysatoren der Alkylenoxid-Gehalt in den nicht-ionogenen Tensiden auf unter 1 ppm und der Dioxan-1,4-Gehalt auf unter 3 ppm senken läßt; außerdem liegt der Polyethylenglycol-Gehalt in den Addukten unter 1 %.

Als Fettalkohol zur Herstellung des Katalysators wird bevorzugt Isotridecanol verwendet, das bevorzugt mit Kalium- oder Natriumhydroxid bei Temperaturen von 100 - 160 °C, bevorzugt 120 - 140 °C, umgesetzt wird.

Die bevorzugt zur Alkoxilierung verwendeten Edukte sind Fettalkohole mit C₄ bis C₂₄ und Alkylphenole mit einem Alkylrest bis C₁₂. Die Temperaturen betragen hierbei etwa 140 - 200 °C, bevorzugt 160 - 190 °C.

Die folgenden Beispiele sollen die Erfindung verdeutlichen.

### Beispiel 1 (erfindungsgemäß)

a) In einem 5-l Glasreaktor mit Heizung, Rührer und Stickstoffeinleitung werden 561 g Kaliumhydroxid (Plätzchen) und 2 029 g i-Tridecanol auf 130 °C unter Rühren aufgeheizt und 48 Stunden bei dieser Temperatur gerührt. Entstehendes Wasser wird mit Hilfe von Stickstoff ausgetrieben.
   Das entstehende Alkoholat ist bei 70 - 90 °C pump- und fließfähig.
b) 561 g 50%ige KOH und 1 014,5 g i-Tridecanol werden bei 60 °C gemischt. Anschließend wird die Emulsion mit 500 g/h Zutropfgeschwindigkeit bei 130 °C unter Stickstoffabdeckung über einen Dünnschichtverdampfer zur Reaktion gebracht und Wasser abgetrennt. Es entsteht ein bei 70 - 90 °C fließ- und pumpfähiges Alkoholat.

### Beispiel 2 (erfindungsgemäß)

a) In der Apparatur und unter den Bedingungen von Beispiel 1a werden 400 g Natriumhydroxid (Plätzchen) und 2 029 g i-Tridecanol vorgelegt. Der Ansatz wird 72 Stunden unter Rühren bei 130 °C belassen. Entstehendes Wasser wird mit Hilfe von Stickstoff ausgetrieben. Das Alkoholat ist bei 70 - 90 °C fließ- und pumpfähig.
b) Am Dünnschichtverdampfer werden unter den Bedingungen von Beispiel 1b aus 800 g 25%iger Natriumhydroxid-Lösung und 1 014,5 g i-Tridecanol ein Natriumisotridecanolat hergestellt, das bei 70 - 90 °C fließ- und pumpfähig ist.

### Beispiel 3 (erfindungsgemäß)

In einem 5-l Reaktor mit Heizung, Rührer und Stickstoffeinleitung werden 720,9 g (3,5 mol) Nonylphenol und 4,08 g (17,12 mmol) K-isotridecanolat auf ca. 60 °C aufgeheizt und unter Stickstoffeinleitung zur Inertisierung verrührt. Anschließend werden bei 180 °C unter 1,5 bar abs Stickstoffvordruck und einem Maximaldruck von 3 bar abs 1 386 g (31,5 mol) Ethylenoxid angelagert.
Rest Ethylenoxid-Gehalt: < 1 ppm
Dioxan-1,4 Gehalt: < 3 ppm

### Beispiel 4 (erfindungsgemäß)

In der Apparatur und unter den Bedingungen von Beispiel 3 werden 689,5 g (3,5 mol) C_{12/18}-Fettalkohol und 1,51 g (6,34 mmol) Na-isotridecanolat auf ca. 60 °C aufgeheizt und unter Stickstoffeinleitung zur Inertisierung verrührt. Anschließend werden bei 160 °C 308 g (7 mol) Ethylenoxid angelagert.
Rest Ethylenoxid-Gehalt: < 1 ppm
Dioxan-1,4 Gehalt : < 3 ppm

### Beispiel 5 (erfindungsgemäß)

In der Apparatur und unter den Bedingungen von Beispiel 3 werden 304,8 g (4 mol) 1,2 Propylenglycol und 79,05 g (0,328 mmol) K-isotridecanolat auf 60 °C aufgeheizt und unter Stickstoffeinleitung zur Inertisierung verrührt. Anschließend werden bei 140 °C 769,9 g (132,5 mol) Propylenoxid angelagert.
Rest Propylenoxid-Gehalt: < 1 ppm

### Beispiel 6 (erfindungsgemäß)

In der Apparatur und unter den Bedingungen von Beispiel 3 werden 689,5 g (3,5 mol) C_{12/14}-Fettalkohol und 1,51 g (6,34 mmol) Na-isotridecanolat auf 60 °C aufgeheizt und unter Stickstoffeinleitung zur Inertisierung verrührt. Anschließend werden bei 160 °C 308 g (7 mol) Ethylenoxid angelagert.
PEG-Gehalt: 0,4 %

### Beispiel 7 (Vergleichsbeispiel)

Wie in Beispiel 3 werden 720,9 g (3,5 mol) Nonylphenol und 2,74 g (17,12 mol) 25%ige NaOH verrührt und Wasser durch Einleitung von Stickstoff bei 130 °C ausgetrieben. Anschließend werden bei 180 °C 1 386 g (31,5 mol) Ethylenoxid angelagert.
Rest Ethylenoxid-Gehalt: 26 ppm
Dioxan-1,4 Gehalt : 118 ppm

### Beispiel 8 (Vergleichsbeispiel)

Wie in Beispiel 5 werden 304,8 g (4 mol) 1,2-Propylenglycol und 0,328 mol Ba-ethylhexanolat, hergestellt nach dem in US-PS 4 453 023 beschriebenen Verfahren, auf 60 °C aufgeheizt und unter Stickstoffeinleitung zur Inertisierung verrührt. Anschließend werden bei 140 °C 7 694,9 g (132,5 mol) Propylenoxid angelagert.
Das hergestellte Polypropylenglycol erreichte nicht die spezifikationsmäßige Viskosität. Das Produkt riecht stark nach Ethylhexanol.

Der Rest Propylenoxid-Gehalt betrug 120 ppm

### Beispiel 9 (Vergleichsbeispiel)

Wie in Beispiel 6 werden 689,5 g (3,5 mol) C_{12/14}-Fettalkohol und 1,51 g (6,34 mmol) Ba-ethylhexanolat, hergestellt nach dem in US 4 453 023-A beschriebenen Verfahren, auf 60 °C aufgeheizt und unter Stickstoffeinleitung zur Inertisierung verrührt. Anschließend werden bei 160 °C 308 g (7 mol) Ethylenoxid angelagert.
PEG-Gehalt: 2,8 %.

### Beispiel 10 (Vergleichsbeispiel)

Wie in Beispiel 4 werden 689,5 g (3,5 mol) C_{12/18}-Fettalkohol und 6,34 mmol Ba-Decanolat, hergestellt nach dem in EP 0 026 547 beschriebenen Verfahren, auf ca. 60 °C aufgeheizt und unter Stickstoffeinleitung zur Inertisierung verrührt. Anschließend werden bei 160 °C 308 g (7 mol) Ethylenoxid angelagert.
Rest Ethylenoxid-Gehalt: 40 ppm
Dioxan-1,4-Gehalt: 110 ppm

## Patentansprüche

1. Verfahren zur Herstellung von alkylenoxid- und dioxan-1,4-armen Alkoxidationsprodukten von Alkylphenolen und Fettalkoholen,
dadurch gekennzeichnet,
daß man als Katalysator durch direkte Umsetzung bei erhöhten Temperaturen von Alkalihydroxid mit Fettalkoholen gewonnene Alkalifettalkoholate einsetzt.

2. Verfahren zur Herstellung von alkylenoxid- und dioxan-1,4-armen Alkoxidationsprodukten von Alkylphenolen und Fettalkoholen nach Anspruch 1,
dadurch gekennzeichnet,
daß man als Alkalihydroxid Natrium- oder Kaliumhydroxid verwendet.

3. Verfahren zur Herstellung von alkylenoxid- und dioxan-1,4-armen Alkoxidationsprodukten von Alkylphenolen und Fettalkoholen nach Anspruch 1,
dadurch gekennzeichnet,
daß man als Fettalkohol zur Katalysatorherstellung Isotridecanol verwendet.

4. Verfahren zur Herstellung von alkylenoxid- und dioxan-1,4-armen Alkoxidationsprodukten von Alkylphenolen und Fettalkoholen nach Anspruch 1,
dadurch gekennzeichnet,
daß man die Umsetzung bei Temperaturen von 100 - 160 °C durchführt.

5. Verfahren zur Herstellung von alkylenoxid- und dioxan-1,4-armen Alkoxidationsprodukten von Alkylphenolen und Fettalkoholen nach Anspruch 4,
dadurch gekennzeichnet,
daß die Temperatur 120 - 140 °C beträgt.

## Claims

1. A process for the preparation of low-alkylene oxide and low-1,4-dioxane alkoxydation products of alkyl phenols and fatty alcohols, characterised in that the catalysts used are alkali metal fatty alcoholates obtained by direct reaction at elevated temperatures of alkali metal hydroxide with fatty alcohols.

2. A process for the preparation of low-alkylene oxide and low-1,4-dioxane alkoxydation products of alkyl phenols and fatty alcohols according to Claim 1, characterised in that the alkali metal hydroxide used is sodium hydroxide or potassium hydroxide.

3. A process for the preparation of low-alkylene oxide and low-1,4-dioxane alkoxydation products of alkyl phenols and fatty alcohols according to Claim 1, characterised in that the fatty alcohol used for the preparation of the catalyst is isotridecanol.

4. A process for the preparation of low-alkylene oxide and low-1,4-dioxane alkoxydation products of alkyl phenols and fatty alcohols according to Claim 1, characterised in that the reaction is carried out at temperatures of from 100 to 160°C.

5. A process for the preparation of low-alkylene oxide and low-1,4-dioxane alkoxydation products of alkyl phenols and fatty alcohols according to Claim 4, characterised in that the temperature is from 120 to 140°C.

## Revendications

1. Procédé de préparation de produits d'alcoxydation d'alkyl-phénols et d'alcools gras pauvres en oxyde d'alkylène et pauvres en dioxanne-(1,4),
caractérisé par le fait que l'on utilise comme catalyseur des alcoolates alcalins gras qui sont obtenus par réaction directe, à des températures élevées, d'un hydroxyde alcalin sur des alcools gras.

2. Procédé de préparation de produits d'alcoxydation d'alkyl-phénols et d'alcools gras pauvres en oxyde d'alkylène et pauvres en dioxanne-(1,4) selon la revendication 1,
caractérisé par le fait que l'on utilise, comme hydroxyde alcalin, l'hydroxyde de sodium ou l'hydroxyde de potassium.

3. Procédé de préparation de produits d'alcoxydation d'alkyl-phénols et d'alcools gras pauvres en oxyde d'alkylène et pauvres en dioxanne-(1,4) selon la revendication 1,
caractérisé par le fait que l'on utilise l'isotridécanol comme alcool gras pour la préparation du catalyseur.

4. Procédé de préparation de produits d'alcoxydation d'alkyl-phénols et d'alcools gras pauvres en oxyde d'alkylène et pauvres en dioxanne-(1,4) selon la revendication 1,
caractérisé par le fait que la réaction est effectuée à des températures de 100 à 160°C.

5. Procédé de préparation de produits d'alcoxydation d'alkyl-phénols et d'alcools gras pauvres en oxyde d'alkylène et pauvres en dioxanne-(1,4) selon la revendication 4,
caractérisé par le fait que la température est de 120 à 140°C.
